# EUROPEAN PATENT APPLICATION

(11) **EP 4 417 704 A1**
(43) Date of publication of application: **21.08.2024**
(21) Application number: 22880745.9
(22) Date of filing: 22.09.2022
(51) Int. Cl.: C12Q 1/6837, G01N 33/574

(54) **METHOD FOR CREATING BIOMARKER SET FOR DETECTING CANCER**

(30) Priority: 15.10.2021 JP 2021169440
(71) Applicant: FUJIFILM Corporation, Tokyo 106-8620 (JP)
(72) Inventor: YAMAGUCHI, Naoko, Ashigarakami-gun, Kanagawa 258-8538 (JP)
(74) Representative: Hughes, Andrea Michelle
(86) International application number: PCT/JP2022/035459
(87) International publication number: WO 2023/063049

(57) **Abstract**

An object of the present invention is to provide a production method of a biomarker set capable of diagnosing the presence or absence of cancer in a plurality of organs in a single cancer screening with higher accuracy than in the related art. The present invention provides a method for producing a biomarker set, the method including a classification step of classifying a plurality of DNA methylation degree data collected according to types of the cancer into one or more groups based on information on tissue type, an extraction step of selecting any one predetermined group from all classified groups, performing two group comparison between the predetermined group and the other group, and extracting information on a DNA position recognized to have a significant difference between both groups and a methylation state of both groups at the position, and a selection step of selecting, from the information on both groups in which the significant difference is recognized, a biomarker which satisfies a predetermined condition, as a biomarker of a cancer type to which the predetermined group belongs.

## Description

### BACKGROUND OF THE INVENTION

### 1. Field of the Invention

The present invention relates to a method for producing a biomarker set used in cancer screening. In particular, the present invention relates to a method for producing a biomarker set for diagnosing whether or not there is a suspicion of cancer (presence or absence of cancer tissue) for a plurality of types of organs at once from a biological specimen such as blood or excrement by distinguishing the types of cancer.

In general, a stepwise examination is required for the definitive diagnosis of the presence or absence of cancer. First, it is diagnosed whether or not there is a suspicion of cancer by cancer screening, and in a case where it is diagnosed that there is a suspicion of cancer, a detailed examination is performed and a definitive diagnosis of the presence or absence of cancer is carried out. In a case where cancer is detected early and treatment is started early, the possibility that the cancer will be completely cured and the survival rate will be increased. Therefore, cancer screening that diagnoses the necessity for a detailed examination is very important. However, there are various problems depending on the type of cancer to be detected in the cancer screening.

For example, in a case where the detection target of cancer is a pancreatic cancer, a liver cancer, or the like, there is a problem that there is no recommended examination because screening examination for which evidence (scientific basis) has been established does not exist yet. In addition, in a case of five cancers recommended to be examined by the guideline of the Ministry of Health, Labour and Welfare, that is, a gastric cancer, a colorectal cancer, a lung cancer, a breast cancer, and a uterine cervical cancer (countermeasure type examination), endoscopy and chest X-ray examination (including mammography) are recommended. However, since a time constraint, an economic burden, and a physical and mental burden are large in these examinations, there is a tendency that the medical examination is avoided.

As a technique that can solve such a problem, liquid biopsy has attracted attention. The liquid biopsy is a technique for detecting a substance (DNA) derived from a cancer cell by using a low-invasive liquid specimen (blood, excrement, or the like) that causes a small burden on a body of a subject at the time of collecting specimen. In this technique, what is called a biomarker is used. The biomarker is a characteristic value for objectively measuring and evaluating a substance derived from a cancer cell, and is used as an indicator for grasping the presence or absence of a disease, the state of progress of the disease, and the like. At present, various biomarkers have been proposed, and research and development are being carried out for practical use. Among these, there is a method for using a methylation state (frequency) of a specific DNA.

Methylation of DNA is largely involved in the control of gene expression, and it is known that methylation or demethylation of a specific methylated site occurs in relation to the onset of cancer. Therefore, in a case where the methylated site specific to the occurrence of the cancer to be examined or the methylation state at the methylated site is clear, it is possible to detect the substance (DNA) derived from a cancer cell in cell-free DNA (cfDNA) extracted from a liquid sample derived from a subject.

In addition, since the positions of the methylated sites (CG sequences) at which methylation and/or demethylation of DNA occurs and the methylation states of these sites, that is, the methylation patterns are different from each other depending on the characteristics such as the types of cancer or the tissue types of cancer, the gender, the age, and the race of the subject, the presence or absence of diseases other than cancers, with which the subject is affected, in a case where a correlation between the types and characteristics of each cancer and the methylation pattern is known, it is possible to create a biomarker specific for the type and characteristics of each cancer based on these information.

For example, JP2019-521673A discloses a method for producing a biomarker based on known data in which a specific type of cancer and a methylation state of DNA thereof are associated with each other in advance.

### SUMMARY OF THE INVENTION

However, in a case where there is no known data that associates the type of cancer with the DNA methylation state, the production method of a biomarker disclosed in JP2019-521673A cannot be used. In such a case, the researchers need to select one to several thousand methylated sites from a wide range of genome regions, or to examine a method for measuring the methylation state or a threshold value to determine the methylation state of DNA, as the biomarker index site. In addition, a biomarker index site that is commonly changed in all the subjects must be selected and determined from a wide range of genome regions and a variety of DNA methylation patterns. That is, there is a problem that it takes a lot of time and cost to acquire a biomarker.

In addition, in a case where a biomarker specific to cancer of each organ is selected and a plurality of selected biomarkers are used in combination as a biomarker set, it is possible to examine the presence or absence of cancer (whether or not there is a suspicion of cancer) of a plurality of organs in one examination. However, there are a plurality of patient groups having different DNA methylation patterns even in a case where the cancer is the same organ, and thus there is a problem in that the accuracy and sensitivity are not sufficient. That is, in general, some of DNA methylation patterns specific to cancer of a predetermined organ are selected to produce a biomarker, but in a case where a subject (patient) having a pattern different from the selected DNA methylation pattern is included in a subject group, there is a problem that the accuracy and sensitivity of the biomarker (and the biomarker set) may be deteriorated depending on the subject (or the subject group).

Therefore, an object of the present invention is to provide a method of easily producing a biomarker set capable of diagnosing the presence or absence of cancer (that is, whether or not there is a suspicion of cancer) in a plurality of organs with higher accuracy (and sensitivity) than in the related art in a single cancer screening even in a case where there are biological specimens derived from a plurality of patient groups having different DNA methylation patterns.
[1] A method for producing a biomarker set, which is a method for producing a biomarker set for use in cancer screening in which cancers of a plurality of types of organs are included as cancer to be diagnosed, the method comprising: a collection step of collecting a plurality of DNA methylation degree data including (1) a position of one or more methylated sites in a whole genome region, (2) methylation degrees of the methylated sites, and (3) information on a tissue type, according to types of the cancer, which are a plurality of known DNA methylation degree data acquired from a biological specimen derived from a patient suffering from the cancer to be diagnosed; a classification step of classifying the plurality of the DNA methylation degree data collected according to the types of the cancer into one or more groups based on the information on the tissue type; an extraction step of selecting any one predetermined group from all classified groups, performing two group comparison between the predetermined group and the other group, and extracting information on a DNA position recognized to have a significant difference between both groups and a methylation state of both groups at the position; a selection step of selecting, from the information on both groups in which the significant difference is recognized, a biomarker which satisfies a predetermined condition, as a biomarker of a cancer type to which the predetermined group belongs; and a step of repeating the extraction step and the selection step until the biomarker is selected for all classified groups, to produce a biomarker set composed of all selected biomarkers, in which the information on the tissue type is included in the DNA methylation degree data of at least one or more types of the cancer.
[2] The method according to [1], in which, in the classification step, in a case where the plurality of the DNA methylation degree data have information on two or more tissue types, the plurality of the DNA methylation degree data are classified into two or more groups, or in a case where the plurality of the DNA methylation degree data have information on only one of the tissue type or have no information on the tissue type, the plurality of the DNA methylation degree data are classified into one group.
[3] The method according to [1] or [2], in which, in the extraction step, determination of the significant difference is performed based on (1) a p-value in a statistical test or (2) a difference between median values or between average values in distribution of the methylation degree.
[4] The method according to any one of [1] to [3], in which an ovarian cancer, a lung cancer, and a liver cancer are included as the cancer to be diagnosed, and the information on the tissue type collected in the collection step is serous carcinoma, clear cell carcinoma, endometrioid carcinoma, and mucinous carcinoma, which are tissue types of the ovarian cancer, adenocarcinoma, squamous cell carcinoma, large cell carcinoma, and small cell carcinoma, which are tissue types of the lung cancer, and hepatocellular carcinoma and intrahepatic cholangiocarcinoma, which are tissue types of the liver cancer.
[5] A method for producing a biomarker set, which is a production method of a biomarker set for producing a biomarker set for use in cancer screening in which cancers of a plurality of types of organs are included as cancer to be diagnosed, the method comprising: a collection step of collecting a plurality of DNA methylation degree data including (1) a position of one or more methylated sites in a whole genome region, (2) methylation degrees of the methylated sites, and (3) information on whether or not suffering from one or more diseases other than cancer, according to types of the cancer, which are a plurality of known DNA methylation degree data acquired from a biological specimen derived from a patient suffering from the cancer to be diagnosed; a classification step of classifying the plurality of the DNA methylation degree data collected according to the types of the cancer into one or more groups based on the information on whether or not suffering from the one or more diseases other than cancer; an extraction step of selecting any one predetermined group from all classified groups, performing two group comparison between the predetermined group and the other group, and extracting information on a DNA position recognized to have a significant difference between both groups and a methylation state of both groups at the position; a selection step of selecting, from the information on both groups in which the significant difference is recognized, a biomarker which satisfies a predetermined condition, as a biomarker of a cancer type to which the predetermined group belongs; and a step of repeating the extraction step and the selection step until the biomarker is selected for all classified groups, to produce a biomarker set composed of all selected biomarkers, in which the information on whether or not suffering from the one or more diseases other than cancer is included in the DNA methylation degree data of at least one or more types of the cancer.
[6] The method according to [5], in which, in the classification step, in a case where the plurality of the DNA methylation degree data have the information on whether or not suffering from the one or more diseases other than cancer, the plurality of the DNA methylation degree data are classified into two or more groups, or in a case where the plurality of the DNA methylation degree data have no information on whether or not suffering from the diseases other than cancer, the plurality of the DNA methylation degree data are classified into one group.
[7] The method according to [5] or [6], in which, in the extraction step, determination of the significant difference is performed based on (1) a p-value in a statistical test or (2) a difference between median values or between average values in distribution of the methylation degree.
[8] The method according to any one of [5] to [7], in which, the information on whether or not suffering from the one or more diseases other than cancer collected in the collection step is whether or not suffering from liver cirrhosis and whether or not suffering from chronic hepatitis, which are highly correlated with the liver cancer.

According to the present invention, even in a case where there are biological specimens derived from a plurality of patient groups having different DNA methylation patterns, it is possible to easily produce a biomarker set capable of diagnosing the presence or absence of cancer (that is, whether or not there is a suspicion of cancer) in a plurality of organs with high accuracy (and sensitivity) for each organ in a single cancer screening.

In addition, since it is possible to detect the presence or absence of a plurality of types of cancer tissues DNA with high accuracy in a single examination, it is possible to reduce a time constraint, an economic burden, and a physical and mental burden on the subject to be examined.

### BRIEF DESCRIPTION OF THE DRAWINGS

Fig. 1 is a flowchart diagram showing an example of a production method of a biomarker set according to the embodiment 1 of the present invention.
Fig. 2A is a diagram for describing an example of a method for comparing between two groups in a selection step of a biomarker of the embodiment 1.
Fig. 2B is a diagram for describing an example of a method for comparing between two groups in a selection step of a biomarker of the embodiment 1.
Fig. 3 is a flowchart diagram showing an example of a production method of a biomarker set according to embodiment 2 of the present invention.
Fig. 4 is a graph showing the results (correct answer rate) of the performance evaluation of the biomarker sets selected in Example 1 and Comparative Example 1.
Fig. 5 is a graph showing the results (correct answer rate) of the performance evaluation of the biomarker sets selected in Example 2 and Comparative Example 1.
Fig. 6 is a graph showing the results (correct answer rate) of the performance evaluation of the biomarker sets selected in Example 3 and Comparative Example 2.
Fig. 7 is a diagram used to describe the term "methylation degree" used in the present invention.

### DESCRIPTION OF THE PREFERRED EMBODIMENTS

Hereinafter, the method for selecting a biomarker according to the present invention will be described in detail based on the preferred embodiment shown in the accompanying drawings.

### (Description of terms)

In the present specification, the term "biomarker" is used for a screening examination for quantifying DNA derived from a cancer tissue of a plurality of types of organs (and visceras) contained in cell-free DNA (cfDNA) by analyzing data of the cell-free DNA (cfDNA) contained in a biological specimen collected from a subject and evaluating whether or not the subject is suffering from cancer, and refers to a methylated site correlated with a specific cancer and a DNA methylation degree at the site. One or more biomarkers are used to determine the presence or absence of the specific cancer.

The term "biomarker set" used in the present invention refers to a combination of one or more biomarkers that detect each cancer type in order to determine the presence or absence of a plurality of types of cancer.

Here, the phrase "screening examination for evaluating whether or not suffering from cancer" using a "biomarker" used in the present specification is a method that includes acquiring a plurality of sequencing reads of cell-free DNA contained in a biological specimen collected from a subject, the sequencing reads each including methylated sequence data (a plurality of methylated sites and a methylation state at the sites) obtained from a continuous nucleic acid sequence of 50 or more nucleic acids, for example, in a case where a next generation sequencer is used, calculating a methylation pattern based on one sequencing read in the plurality of sequencing reads, in which the methylation pattern includes a methylation state of a genome region corresponding to the continuous nucleic acid sequence and one or more motifs within the genome region (that is, a biologically meaningful base sequence pattern of approximately 5 to 20 base pairs), and calculating one or more likelihood scores by comparing the methylation pattern with each of one or more biomarkers (that is, the plurality of the methylated site and the methylation state thereof), in which each of the one or more biomarkers is correlated with specific cancer tissue DNA, and each of the biomarkers includes at least one predetermined methylated site and a methylation state at the site, the sequencing read is evaluated as including cancer tissue in a case where at least one of the one or more likelihood scores exceeds a threshold value, and specific cancer tissue in the biological specimen is quantitatively evaluated based on the number of sequencing reads including "cancer tissue" in the plurality of sequencing reads.

In addition, the phrase "screening examination for evaluating whether or not suffering from cancer" using the "biomarker" used in the present specification is not limited to the examination using the next generation sequencer, and for example, a method of directly acquiring the methylation degree of a plurality of methylated sites of cell-free DNA contained in a biological specimen collected from a subject using a method such as a microarray, and performing evaluation as including the predetermined cancer tissue DNA (DNA in a predetermined methylation state) in the biological specimen in a case where the methylation degree at a predetermined one or more methylated sites exceeds or does not exceed a predetermined threshold value correlated with a predetermined prescribed cancer tissue.

Furthermore, it is an examination capable of examining the presence or absence of cancer (that is, whether or not there is a suspicion of cancer) for a plurality of organs at once by using a biomarker set consisting of biomarkers different depending on the cancer type.

The term "methylated site" used in the present specification refers to a cytosine residue (CpG site) adjacent to a guanine residue, which can be methylated.

The term "methylation pattern" used in the present specification refers to a DNA methylation pattern characteristic of a predetermined physiological composition, and refers to a combination of at least one or more methylated sites and a methylation degree of the sites.

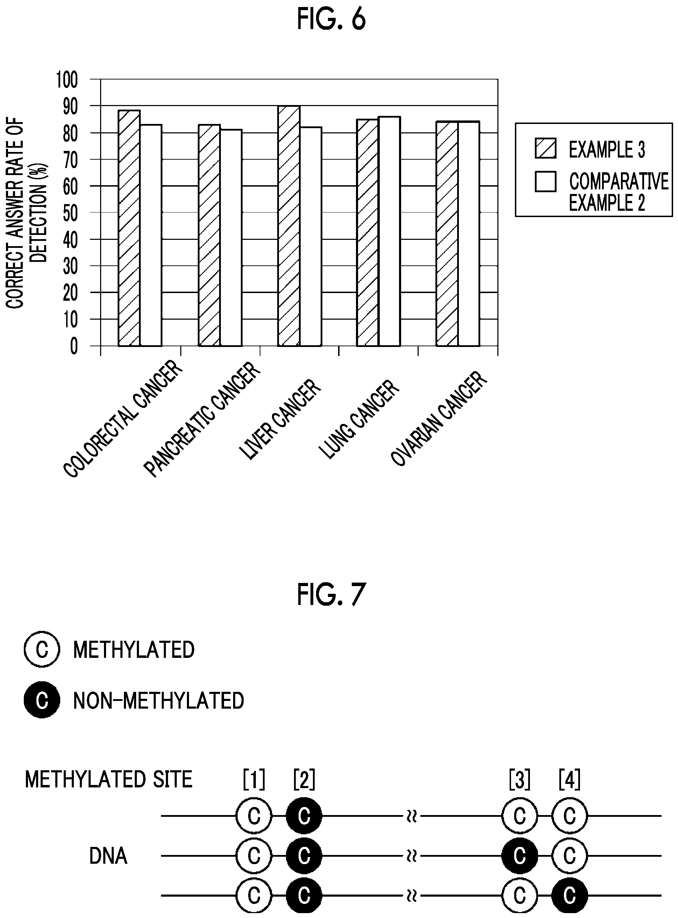

The term "methylation degree" used in the present invention refers to a proportion of DNA that is methylated with respect to all DNA at a position of a methylated site (CpG site) common to a plurality of (a plurality of or the same cell-derived) DNAs. As shown in Fig. 7, in a case where there are three copies of DNA, the methylation degrees of the CpG sites [1] to [4] are each 100% for the CpG site [1], 0% for the CpG site [2], and 66.7% for the CpG sites [3] and [4].

The term "methylation state" used in the present invention refers to information on whether distribution of a methylation degree of a certain group is high (methylated) or low (non-methylated) at a predetermined methylated site.

The phrase "cancer to be detected" used in the present specification refers to a cancer (to be diagnosed) of an organ to be targeted in an initial examination (an examination for determining whether or not a detailed examination is necessary) recommended for the purpose of early detection, early treatment, and appropriate treatment. Examples thereof include cancerous cells in a tongue, an esophagus, a lung, a gastrointestinal tract, a pancreas, a kidney, a liver, a breast, a prostate, a uterus, a bladder, an ovary, blood, a brain, and the like, or cancerous cells derived from these. The cancer may include a tumor composed of tumor cells.

The number of types of cancers of the target organ to be detected by the biomarker set according to the embodiment of the present invention is not particularly limited as long as it is at least two or more, and is preferably 5 or more and more preferably 10 or more.

The term "accuracy" used in the present specification refers to a degree of accuracy in correctly determining a person who has a cancer as positive and correctly determining a person who does not have a cancer as negative.

The term "sensitivity" used in the present specification refers to a proportion of a person who is found to have a cancer by an examination among persons who have a cancer that can be found at the time of the medical examination.

The term "biological specimen" used in the present specification refers to a specimen collected from a body of a subject, and specifically refers to blood, skin, hair, saliva, oral mucous, vaginal mucous, sweat, tears, epithelial tissue, urine, semen, seminal plasma, a prostatic fluid, excrement, biopsy, ascites, a cerebrospinal fluid, a lymphatic fluid, and a tissue extract specimen or a biopsy specimen, which are collected from a subject and having a contact with one or more organs (visceras).

### [Embodiment 1]

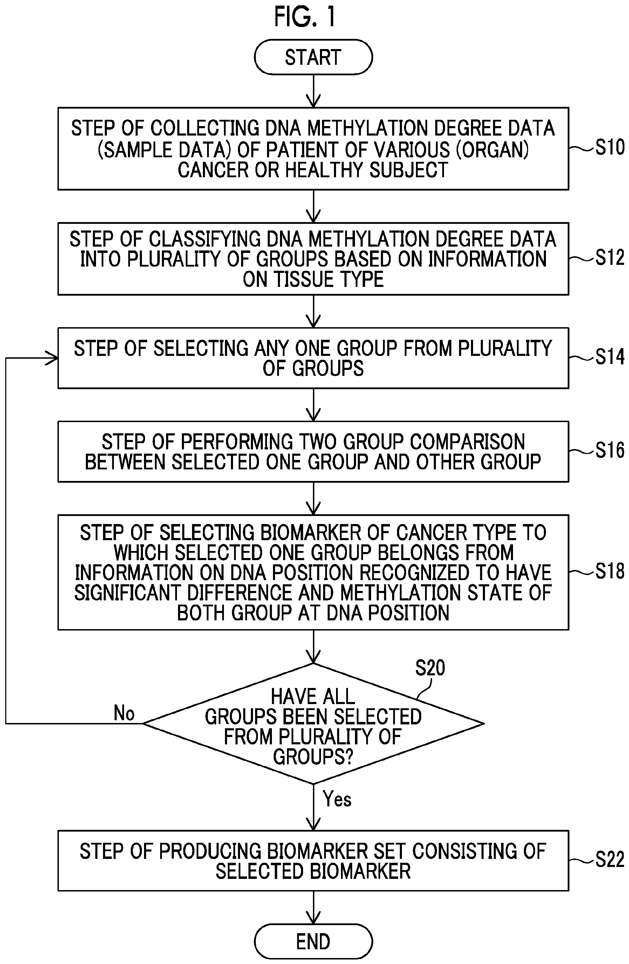

Fig. 1 conceptually shows a flowchart of an example of a production method of a biomarker set according to the embodiment 1 of the present invention.

### (Collection step of DNA methylation degree data)

As shown in Fig. 1, first, a sample of a patient suffering from various cancers (and a healthy subject) is acquired by using a publicly available database, and DNAmethylation degree data of a cancer tissue to be detected (and a normal tissue) is collected from the sample (Step S10). The DNA methylation degree data includes at least three types of information, that is, (1) a position of one or more methylated sites in the entire genome region, (2) methylation degrees of the methylated sites, and (3) information on a tissue type.

The number of samples of the patient (or the healthy subject) for each cancer tissue (or normal tissue) is not particularly limited as long as there are at least 5 samples, and is preferably 100 or more and more preferably 200 or more.

The phrase "position of one or more methylated sites in the entire genome region" refers to the positions of tens of thousands to hundreds of thousands of cytosine bases obtained by genome-wide measurement such as microarray or whole genome sequencing, that is, comprehensive measurement of all regions of the genome, and for example, refers to the positions of all cytosine residues measured by microarray of Illumina, Inc. such as Infinium Human Methylation 450, whole genome bisulfite sequencing (WGBS), and reduced representation of bisulfite sequencing (RRBS).

The information about the tissue type is acquired from the information associated with the sample. Generally, information on the gender or age of a patient, or the stage or tissue type of cancer is attached to the sample. The tissue type is a classification of cancer based on the shape of cancer cells or the state of a tissue in which cancer cells are collected. The information on a tissue type is not particularly limited as long as information on at least one type of cancer among a plurality of cancers to be detected (organs) can be acquired, but preferably, it is preferable to collect the information on the tissue type of all types of cancer to be detected.

For example, in a case where the examination target is a liver cancer, examples of the tissue type include hepatocellular carcinoma (HCC), intrahepatic cholangiocarcinoma (ICC), and a mixed type of HCC and ICC. In addition, in a case where the examination target is a lung cancer, examples of the tissue type include squamous cell carcinoma, lung adenocarcinoma, lung large cell carcinoma, and non-small cell carcinoma. Furthermore, in a case where the examination target is an ovarian cancer, examples of the tissue type include serous carcinoma, clear cell carcinoma, endometrioid carcinoma, and mucinous carcinoma.

The public database is not particularly limited as long as whole genome bisulfite sequencing (WGBS), reduced representation of bisulfite sequencing (RRBS), and DNA methylation degree data based on an array are included, and for example, data analyzed in a large-scale cancer genome project The Cancer Genome Atlas (TCGA) started in the United States from 2006, or microarray data registered in Gene Expression Omnibus (GEO) as a supplement to papers related to DNA methylation, which are publicly available in National Center for Biotechnology Information (NCBI), can be used. The methylation degree is shown by a value of 0 (non-methylated) to 1 (completely methylated).

The collection of the DNA methylation degree data is not limited to the collection from the public database, and an original database uniquely constructed by acquiring a biological specimen from a cancer patient and performing a microarray analysis may be used.

### (Classification step of data)

Next, a plurality of DNA methylation degree data collected for each cancer type are classified into one or more groups based on the information on the "tissue type" of the DNA methylation degree data acquired in the previous step (Step S12).

More specifically, the plurality of DNA methylation degree data of the cancer types having the information on the "tissue type" are divided in a tissue type and classified into one or more groups, and the plurality of DNA methylation degree data of the cancer types having the information on the "tissue type" and the plurality of DNA methylation degree data of the normal cells are not classified and are treated as one group.

By performing such classification, even in a case where there are biological specimens derived from a plurality of patient groups having different DNA methylation patterns, it is possible to easily produce a biomarker set capable of diagnosing the presence or absence of cancer (that is, whether or not there is a suspicion of cancer) in a plurality of types of organs with higher accuracy than in the related art in a single cancer screening. More specifically, in the single cancer screening for examining the presence or absence of cancer in a plurality of types of organs, it is possible to easily acquire a biomarker capable of accurately detecting tissue DNA of a cancer type having information on a "tissue type" in DNA methylation degree data and furthermore it is possible to acquire a biomarker capable of accurately detecting tissue DNA of a cancer type not having information on a "tissue type" in DNA methylation degree data with the same or higher accuracy than that in the related art.

### (Selection step of biomarker)

Next, any one group is selected from among one or more groups classified in the classification step (Step S14), and the two group comparison between the selected group and the remaining group (other group) is performed at a position of DNA common to both groups (Step S16).

By this comparison, the DNA position in which a statistically significant difference is recognized and the methylation states of both groups at the DNA positions (that is, the methylation degree indicating the methylated/non-methylated) are extracted. Here, in a case where the DNA position in which a significant difference is recognized and the methylation state of both groups at the DNA position are extracted, it is considered that both groups can be distinguished and determined.

Then, at least one DNA position and a methylation state of the selected group at the DNA position are selected in order of a large significant difference as a biomarker for detecting a cancer type (organ) to which the selected group belongs from among one or more groups classified in the classification step (Step S 18).

It is preferable that, as the numerical range of the selected biomarker, the lower limit is determined to have redundancy in consideration of biological noise and measurement noise, and the upper limit is determined in consideration of the number of samples of the selected group. Generally, the number of biomarkers of the selected group is selected not to exceed the number of samples of the selected group.

The number of biomarkers to be selected here is not limited as long as it is 1 or more, but is preferably 5 to 100 and more preferably 10 to 30.

Here, a method of the two group comparison in Step S16 will be described.

### (1) Method using statistical hypothesis test

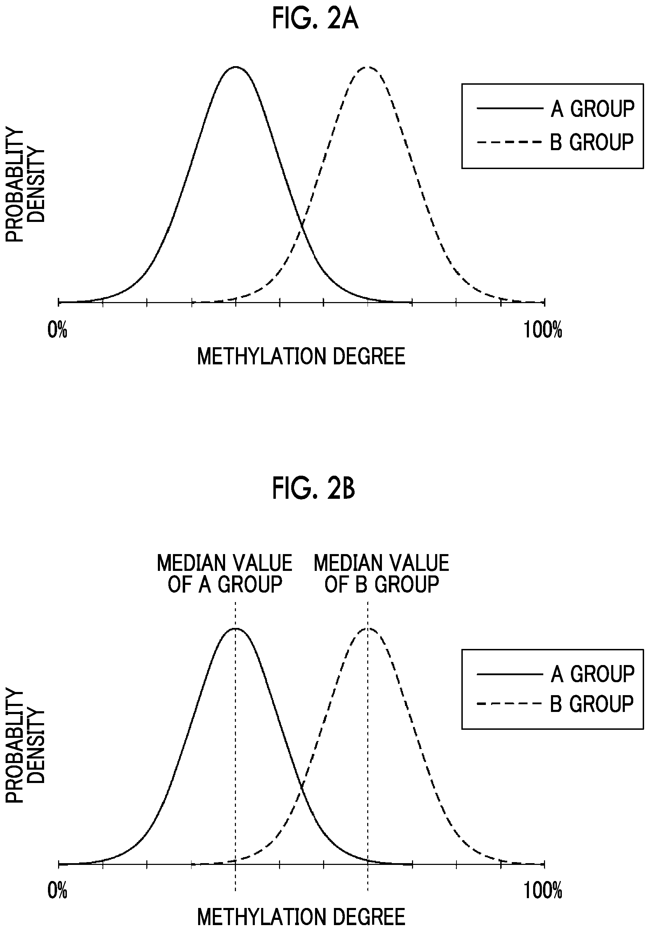

For example, in a case of testing whether or not there is a significant difference between the DNA methylation degree data of the A group (for example, 200 persons) and the DNA methylation degree data of the B group (for example, 100 persons), the distribution of the methylation degree of the A group and the distribution of the methylation degree of the B group are compared at the DNA position common to both groups. The deviation of both distributions can be determined by using a statistical test. First, as shown in Fig. 2A, the distribution is visualized in advance, and a suitable test method [1] to [3] is selected according to the shape of the distribution.
[1] In a case where the distribution is considered to be a normal distribution and the variances of the two distributions are equal, Student's t-test is selected.
[2] In a case where the distribution is considered to be a normal distribution and the variances of the two distributions are not equal, Welch's t-test is selected.
[3] In a case where the distribution is not considered to be a normal distribution, the Mann-Whitney's U-test is selected.

In these tests, in a case where the p-value is 0.05 or less, it is determined that the distributions of two methylation degree are deviated with each other, that is, a significant difference is recognized, and it is considered that the A group and the B group can be distinguished from each other.

In the present embodiment, as a result of performing such a comparison, the DNA positions in which a statistically significant difference is recognized and the methylation states of both groups at the DNA positions (that is, the methylation degree indicating the methylated/non-methylated) are arranged in order of small p-values.

Then, at least one DNA position and a methylation state of the selected group at the DNA position are selected in order of small p-values as a biomarker for detecting a cancer type (organ) to which the selected group belongs from among one or more groups classified in the classification step (Step S18).

For example, in the classification step, in a case where all collected methylation degree data are classified into groups A to D and the A group is arbitrarily selected from the groups A to D, and information on the DNA position having a p-value of ≤0.05 at which both groups can be distinguished in a case where two group comparison is performed in a combination of A × (B + C + D) (in Fig. 2A, B + C + D corresponds to "group B" in the figure), and the methylation state of both groups at the DNA position can be obtained.

Next, information on one or more DNA positions and a methylation state of the selected A group at the DNA position are selected in order of a small p-value from the acquired information, and these information are selected as a biomarker for detecting the tissue DNA of the cancer type (organ) to which the A group belongs.

The two group comparison may be performed by a combination of A × B, A × C, and A × D. In this case, information on each of the DNA position having a p-value of ≤0.05, which is considered that both groups can be distinguished and determined, and the methylation state of both groups (that is, A and B, A and C, and A and D) at the DNA position is obtained.

Next, information on one or more DNA positions and the methylation state of the selected A group at the DNA position are selected in order of small maximum value of p-value (maximum value of A × B, A × C, and A × D) from the acquired information on the methylation state, and these information are selected as a biomarker for detecting the tissue DNA of the cancer type (organ) to which the A group belongs. However, it is not always necessary to perform the two group comparison for the group belonging cancer type to which the A group belongs, that is, the group belonging to the same cancer type.

### (2) Method using representative value

In addition, the deviation of both distributions of the A group and the B group can be determined by using a method in which a representative value of the distribution of the methylation degree is used. First, representative values (a median value or an average value) of the distribution of the methylation degree of both two groups are calculated, and in a case where there is a deviation of at least 30% or more in the calculated values of both groups, it is determined that the distributions of the methylation degree of both groups are deviated with each other, that is, a significant difference is recognized, and thus the A group and the B group can be distinguished and determined.

For example, first, as shown in Fig. 2B, in a case of testing whether or not there is a significant difference between the DNA methylation degree data of the A group and the DNA methylation degree data of the B group, at a DNA position common to both groups, a median value of the distribution of the methylation degree of the A group (for example, 200 persons) and a median value of the distribution of the methylation degree of the B group (for example, 100 persons) are calculated. In a case where the median values of both groups are compared and at least 30% or more of the deviation is present, it is determined that the distributions of the methylation degree of both groups are deviated with each other, and it is considered that the A group and the B group can be distinguished and determined.

Similarly, in a case where the average values of the distributions of the methylation degree of the A group and the B group are calculated and compared with each other and at least 30% or more of the deviation is present, it can be determined that the two methylation degree distributions are deviated with each other.

In Fig. 2B, for example, in the classification step, all collected methylation degree data is classified into groups A to D, and in a case where the A group is arbitrarily selected from the groups A to D, B + C + D corresponds to a "B group" in the figure.

As described above, in a case where the selection of the biomarker based on one group is completed, in Step S14, from a plurality of the groups, it is determined whether or not the selection of all the groups is completed, that is, whether or not the selection work of the biomarker of all the groups is completed (Step S20). In a case where the selection of all the groups is completed, the process proceeds to the next step (Step S22), and in a case where the selection of all the groups is not completed, the step of selecting the biomarker is repeated until the selection is completed (Steps S14 to S20).

### (Production of biomarker set)

In a case where the selection work of the biomarkers in all the groups is completed, a biomarker set using all the selected biomarkers is produced in each group (Step S22).

The number of biomarkers constituting the biomarker set is not particularly limited as long as it is 2 or more, but is preferably 10 to 1,000 and more preferably 30 to 300.

The number of biomarkers may be the same or different between the cancer types, but it is preferable that the number of biomarkers is the same between the groups.

The difference in the number of biomarkers between the groups is not particularly limited, but is preferably ±0% to ±300% and more preferably ±0% to ±50% of the number of selected biomarkers.

As described above, in the biomarker set produced by the method according to the present embodiment 1, it is possible to detect whether or not the cancer tissue DNA is present in the cell-free DNA contained in the biological specimen collected from the subject with the same as or higher accuracy than that of the related art for each organ.

In addition, it is also possible to determine which tissue type cancer of the organ belongs to by performing an examination of whether or not the methylation pattern of the cell-free DNA contained in the biological specimen matches the methylation pattern of the biomarker selected by the method according to the present embodiment 1.

Furthermore, since it is possible to detect the presence or absence of a plurality of types of cancer tissues DNA in a single examination, it is possible to reduce a time constraint, an economic burden, and a physical and mental burden on the subject to be examined.

### [Embodiment 2]

The DNA methylation degree data collected for each cancer to be detected is classified based on the information on the "tissue type" in the embodiment 1, but the present invention is not limited thereto, and the DNA methylation degree data can be classified based on the "whether or not suffering the disease other than cancer".

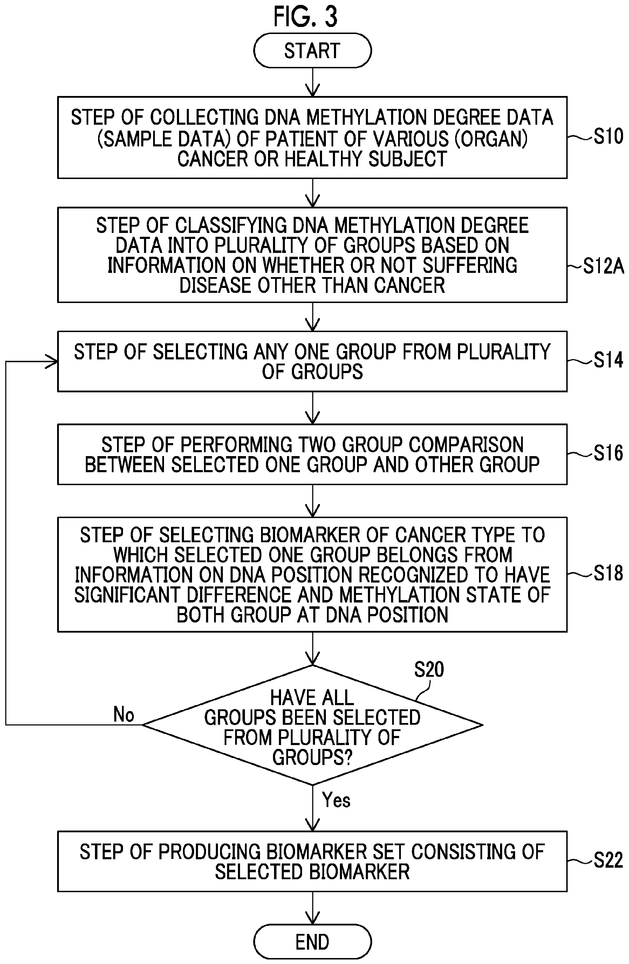

A production method of a biomarker set according to an embodiment 2 shown in Fig. 3 has the same steps as those of the production method of a biomarker set according to the embodiment 1 shown in Fig. 1, except for the classification step (Step S12A) of data. Therefore, the same steps are denoted by the same reference numerals, and the description thereof will be omitted.

In the collection step (Step S10) of DNA methylation degree data, information on the "whether or not suffering the disease other than cancer" is acquired instead of the information on the "tissue type".

The information on whether or not suffering the disease other than cancer is acquired from the information associated with the sample. The information on whether or not suffering the disease other than cancer is not particularly limited as long as the information on whether or not suffering the diseases other than cancer, which is associated with the sample of at least one type of the cancer to be detected among the plurality of cancer to be detected (organs) can be acquired. However, it is preferable to collect the information on the presence or absence of the diseases other than cancer, which is associated with the sample of all types of cancer to be detected. For example, in a case where the detection target is cancer of the organs A to D, the information on whether or not suffering the disease other than cancer is not particularly limited as long as the information on the presence or absence of the diseases other than cancer, which is associated with the sample derived from the patient of at least the organ A, can be acquired.

In addition, it is preferable to collect two or more information on "whether or not suffering the disease other than cancer" correlated with the cancer to be detected. For example, the number of diseases other than cancer, which is associated with the sample derived from a cancer patient of the organ A, is not particularly limited, but is preferably 2 or more.

The "disease other than cancer" is not particularly limited as long as it is a disease provided as information attached to the sample, but a disease correlated with various cancers is preferable. Examples of the disease having a correlation include the following diseases.

In a case where the cancer to be detected is "liver cancer", examples of the disease other than cancer include liver cirrhosis, chronic hepatitis, alcoholic liver disease, and drug-induced liver disease.

In a case where the detection target is "colorectal cancer", examples of diseases other than cancer include ulcerative colitis, Crohn's disease, ischemic colitis, and drug-induced colitis.

In a case where the detection target is "pancreatic cancer", examples of diseases other than cancer include chronic pancreatitis.

In a case where the detection target is "lung cancer", examples of diseases other than cancer include chronic obstructive pulmonary disease.

In a case where the detection target is "ovarian cancer", examples of diseases other than cancer include uterine adnexitis.

Then, in the classification step (Step S12A), classification is performed by based on the "whether or not suffering the disease other than cancer" instead of the "tissue type".

In a case where the plurality of DNA methylation degree data have information on whether or not suffering one or more diseases other than cancer, the plurality of DNA methylation degree data can be classified into two or more groups, and in a case where the plurality of DNA methylation degree data have no information on whether or not suffering the disease other than cancer, the plurality of DNA methylation degree data can be classified into one group.

By performing such classification, even in a case where there are biological specimens derived from a plurality of patient groups having different DNA methylation patterns, it is possible to easily produce a biomarker set capable of diagnosing the presence or absence of cancer (that is, whether or not there is a suspicion of cancer) in a plurality of types of organs with higher accuracy than in the related art in a single cancer screening. More specifically, in a single cancer screening for examining the presence or absence of cancers in a plurality types of organs, it is possible to acquire a biomarker capable of accurately detecting tissue DNA of a cancer type having information on "whether or not suffering one or more diseases other than cancer" in DNA methylation degree data, and it is possible to easily acquire a biomarker capable of detecting tissue DNA of a cancer type having no information on "whether or not suffering one or more diseases other than cancer" in DNA methylation degree data with the same or higher accuracy than that in the related art.

As described above, in the biomarker set created by the method according to the present embodiment 2, it is possible to detect whether or not the cancer tissue DNA is present in the cell-free DNA contained in the biological specimen collected from the subject the same as or higher accuracy than that of the related art for each organ.

In addition, since it is possible to detect the presence or absence of a plurality of types of cancer tissues DNA with high accuracy in a single examination, it is possible to reduce a time constraint, an economic burden, and a physical and mental burden on the subject to be examined.

Although the present invention has been described in detail above, the present invention is not limited to the embodiment described above, and it is needless to say that various improvements or changes may be made without departing from the gist of the present invention. Examples

### [Example 1]

### (Collection step of methylation degree data)

As shown in Table 1, DNA methylation degree data (sample) derived from each of (1) a colorectal cancer tissue, (2) a pancreatic cancer tissue, (3) a liver cancer tissue, (4) a lung cancer tissue, (5) an ovarian cancer tissue, and (6) a non-cancer tissue (normal leukocyte) was collected from TCGA and/or GEO of NCBI. The DNA methylation degree data consists of information on "position of one or more methylated sites in the entire genome region", "methylation degree of the methylated site", and the tissue type of ovarian cancer. The cancer tissue DNAs (1) to (5) of each organ in which cancer has occurred are samples derived from each cancer patient, and the non-cancer tissue DNA (6) is a sample derived from a healthy subj ect.

It is noted that all of the collected data were measured using the microarray Infinium Human Methylation 450 (manufactured by Illumina, Inc.). This microarray can measure the methylation degree of about 450,000 cytosines. In the present examples, all cytosine bases measured by Infinium Human Methylation 450 and the methylation degree of these cytosine bases were used for the "position of one or more methylated sites in the entire genome region" and the "methylation degree of the methylated site" of the DNA methylation degree data.

### (Classification step of data)

Next, regarding the sample data of (5) the ovarian cancer, the sample of (5) ovarian cancer tissue DNA was further subdivided and classified into the serous carcinoma, the clear cell carcinoma, the endometrioid carcinoma, and the mucinous carcinoma based on the information on the tissue type, and the other cancers were not classified. As a result, the group of the cancer tissue DNA of the organ in which cancer occurred and the like (1) to (6) was subdivided into a total of nine groups (A to I). The number of samples in each group is as shown in Table 1.

The DNA methylation data (sample) of each group was randomly divided into two equal parts, one sample group was used as training data of a machine learning model used for selection of a biomarker, production of a set of the biomarkers, and performance evaluation of the produced biomarker set, and the other sample group was used for performance evaluation of the produced biomarker.

**[Table 1]**

| Organ in which cancer occurred (cancer tissue DNA) | Database/GEO number | Classification based on tissue type | Number of samples | Group |
|---|---|---|---|---|
| (1) Colorectal cancer tissue DNA | TCGA | - | 408 | A |
| (2) Pancreatic cancer tissue DNA | TCGA | - | 173 | B |
| (3) Liver cancer tissue DNA | TCGA GEO(GSE32079) | - | 460 | C |
| (4) Lung cancer tissue DNA | TCGA | - | 832 | D |
| (5) Ovarian cancer tissue DNA | TCGA | Serous carcinoma | 63 | E |
| | | Clear cell carcinoma | 13 | F |
| | | Endometrioid carcinoma | 11 | G |
| | GEO(GSE51820) | | | |
| | | Mucinous carcinoma | 8 | H |
| (6) Normal leukocyte DNA | GEO(GSE87571) | - | 732 | I |

### (Selection step of biomarker and production step of biomarker set)

Next, in order to extract "the position and the methylation degree of DNA" that allow A group and the other group (B group to I group) to be distinguished from each other, a methylation degree distribution at a DNA position common to each group was created. That is, the methylation degree distribution of the A group, the methylation degree distribution of the B group, ... and the methylation degree distribution of the I group at the predetermined position of the collected DNA were created at all positions of the collected DNA. Since any of the methylation degree distributions could be regarded as a normal distribution, and it could be determined that the variances of all the distributions were the same, Student's t-test was used for the selection of the biomarker.

Subsequently, in order to select a biomarker capable of distinguishing the sample (A) of the colorectal cancer group from the samples (B to I) of the cancer group of other organs and the normal leukocyte group, a p-value for the null hypothesis that "there is no difference in the average value between the two groups" was calculated between the A group and each other sample to be distinguished (that is, between the A group and the B group + the C group + ... + the I group) at all positions of the DNA.

14 "position of DNA and methylation degree of DNA at the position (methylation degree of A group)" were selected in order of smaller p-values from the "the position and the methylation degree of DNA" satisfying calculated p-values ≤ 0.05, and the selected 14 "position of DNA and methylation degree of DNA at the position (methylation degree of A group)" were selected as biomarkers for detecting whether or not the colorectal cancer tissue DNA to which the A group belongs is present.

In the calculation of the p-value, a programming language for statistical analysis called R language and a t.test function of R language were used.

In the other groups (B to I) as in the A group, the p-value was calculated at all positions of DNA, 14 "position of DNA and methylation degree of DNA at the position" were selected in order of smaller p-values from the "the position and the methylation degree of DNA" satisfying the p-values ≤ 0.05, and the selected 14 "position of DNA and methylation degree of DNA at the position" were selected as the biomarkers for detecting whether or not the cancer tissue DNA to which each group belongs is present. The "position of DNA and methylation degree of DNA of A group" selected in the A group is selected as a biomarker for detecting whether or not the colorectal cancer tissue DNA is present, the "position of DNA and methylation degree of DNA of B group" selected in the B group is selected as a biomarker for detecting whether or not the pancreatic cancer tissue DNA is present, the "position of DNA and methylation degree of DNA of C group" selected in the C group is selected as a biomarker for detecting whether or not the liver cancer tissue DNA is present, the "position of DNA and methylation degree of DNA of D group" selected in the D group is selected as a biomarker for detecting whether or not the lung cancer tissue DNA is present, the "position of DNA and methylation degree of DNA of E group to H group" selected in the E group to H group is selected as a biomarker for detecting whether or not the ovarian cancer tissue DNA is present, the "position of DNA and methylation degree of DNA of I group" selected in the I group is selected as a biomarker for detecting whether or not the normal leukocyte DNA is present.

126 biomarkers of 14 pieces/group × 9 groups = 126 pieces selected in this way was produced as a biomarker set for examining the presence or absence of cancer in a plurality of types of organs at once.

### (Performance evaluation of biomarker set)

First, the machine learning model SVM was trained with the sample group used for the selection of the biomarker (that is, the methylation degree data group used for the selection of the 126 biomarkers), and then it was estimated which organ the cancer tissue DNA of each methylation degree data of the sample group not used for the selection of the biomarker was derived from among (1) colorectal cancer tissue, (2) pancreatic cancer tissue, (3) liver cancer tissue, (4) lung cancer tissue, and (5) ovarian cancer tissue (R language and the svm function in the library e1071 of R language were used for the learning and estimation). The performance evaluation was shown by the estimated correct answer rate for each cancer type.

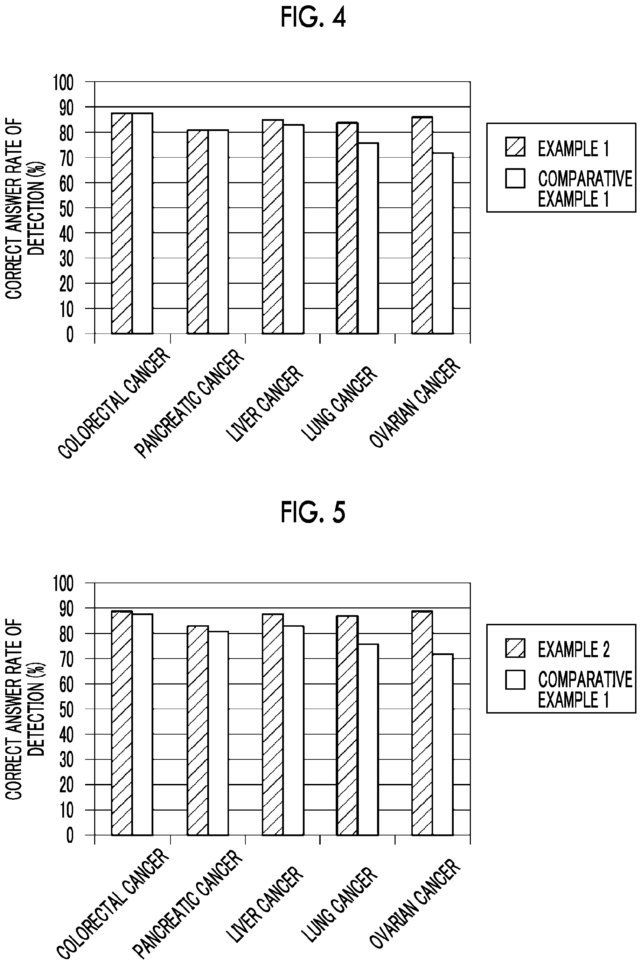

The correct answer rates of the biomarker sets selected in Example 1 were 88% for colorectal cancer, 81% for pancreatic cancer, 85% for liver cancer, 84% for lung cancer, and 86% for ovarian cancer (see Fig. 4).

### [Example 2]

### (Collection step of methylation degree data)

In the same manner as in Example 1, DNA methylation degree data (sample) derived from each of (1) a colorectal cancer tissue, (2) a pancreatic cancer tissue, (3) a liver cancer tissue, (4) a lung cancer tissue, (5) an ovarian cancer tissue, and (6) a non-cancer tissue (normal leukocyte) was collected from TCGA and/or GEO of NCBI (see Table. 2). The DNA methylation degree data consists of a position of one or more methylated sites in the entire genome region, a methylation degree of the methylated site, and information on a tissue type of (3) a liver cancer, (4) a lung cancer, and (5) an ovarian cancer. In the same manner as in Example 1, all cytosine bases measured by Infinium Human Methylation 450 and the methylation degree of these cytosine bases were used for the genome region of the DNA methylation degree data and the methylation state of the genome region.

### (Classification step of data)

Next, for the sample data of (3) a liver cancer, (4) a lung cancer, and (5) an ovarian cancer, based on information on the tissue type, the sample of (3) liver cancer tissue DNA was further classified into hepatocellular carcinoma and intrahepatic cholangiocarcinoma, the sample of (4) lung cancer tissue DNA was further classified into adenocarcinoma and squamous cell carcinoma, and the sample of (5) ovarian cancer tissue DNA was classified into serous carcinoma, clear cell carcinoma, endometrioid carcinoma, and mucinous carcinoma. On the other hand, the other cancers that have no information on the tissue type were not classified. As a result, the group of cancer tissue DNAs of the organ in which cancer occurred (1) to (6) was subdivided into a total of 11 groups of A to K. The number of samples in each group is as shown in Table 2.

The DNA methylation data (sample) of each group was randomly divided into two equal parts, one sample group was used as training data of a machine learning model used for selection of a biomarker, production of a set of the biomarkers, and performance evaluation of the produced biomarker set, and the other sample group was used for performance evaluation of the produced biomarker.

**[Table 2]**

| Organ in which cancer occurred (cancer tissue DNA) | Database/GEO number | Classification based on tissue type | Number of samples | Group |
|---|---|---|---|---|
| (1) Colorectal cancer tissue DNA | TCGA | - | 408 | A |
| (2) Pancreatic cancer tissue DNA | TCGA | - | 173 | B |
| (3) Liver cancer tissue DNA | TCGA | Hepatocellular carcinoma | 375 | C |
| | GEO(GSE32079) | Intrahepatic cholangiocarcinoma | 85 | D |
| (4) Lung cancer tissue DNA | TCGA | Adenocarcinoma | 468 | E |
| | | Squamous cell carcinoma | 364 | F |
| (5) Ovarian cancer tissue DNA | TCGA | Serous carcinoma | 63 | G |
| | | Clear cell carcinoma | 13 | H |
| | | Endometrioid carcinoma | 11 | I |
| | GEO(GSE51820) | | | |
| | | Mucinous carcinoma | 8 | J |
| (6) Normal leukocyte DNA | GEO(GSE87571) | - | 732 | K |

### (Selection step of biomarker and production step of biomarker set)

In the same manner as in Example 1, 12 biomarkers were selected for one group from the collected DNA methylation data. The "position of DNA and methylation degree of DNA of A group" selected in the A group is selected as a biomarker for detecting whether or not the colorectal cancer tissue DNA is present, the "position of DNA and methylation degree of DNA of B group" selected in the B group is selected as a biomarker for detecting whether or not the pancreatic cancer tissue DNA is present, the "position of DNA and methylation degree of DNA of C group and D group" selected in the C group and D group is selected as a biomarker for detecting whether or not the liver cancer tissue DNA is present, the "position of DNA and methylation degree of DNA of E group and F group" selected in the E group and F group is selected as a biomarker for detecting whether or not the lung cancer tissue DNA is present, the "position of DNA and methylation degree of DNA of G group to J group" selected in the G group to J group is selected as a biomarker for detecting whether or not the ovarian cancer tissue DNA is present, the "position of DNA and methylation degree of DNA of K group" selected in the K group is selected as a biomarker for detecting whether or not the normal leukocyte DNA is present.

In addition, 132 biomarkers of 12 pieces/group × 11 groups = 132 pieces were produced as a biomarker set for examining the presence or absence of cancer in various types of organs at once.

### (Performance evaluation of biomarker set)

In the same manner as in Example 1, after training the machine learning model SVM with the sample group used for the selection of the biomarker (that is, the methylation degree data group used for the selection of 132 biomarkers), it was estimated which organ the cancer tissue DNA of each methylation degree data of the sample group not used for the selection of the biomarker was derived from among (1) a colorectal cancer tissue, (2) a pancreatic cancer tissue, (3) a liver cancer tissue, (4) a lung cancer tissue, and (5) an ovarian cancer tissue. In the same manner as in Example 1, the performance evaluation was shown by the estimated correct answer rate for each cancer type.

The correct answer rates of the biomarker sets selected in Example 2 were 89% for colorectal cancer, 83% for pancreatic cancer, 88% for liver cancer, 87% for lung cancer, and 89% for ovarian cancer (see Fig. 5).

### [Comparative Example 1]

### (Collection step of methylation degree data/classification step of data)

In the collection of the methylation degree data, the collection step of the methylation degree data and the classification step of data were performed in the same manner as in Examples 1 and 2, except that the information on the tissue type was not acquired in any cancer type. As a result, the group of cancer tissue DNAs of the organ in which cancer occurred (1) to (6) was classified into a total of 6 groups A to F without being subdivided. The number of samples in each of the groups A to F is as shown in Table 3.

**[Table 3]**

| Organ in which cancer occurred (cancer tissue DNA) | Database/GEO number | Classification based on tissue type | Number of samples | Group |
|---|---|---|---|---|
| (1) Colorectal cancer tissue DNA | TCGA | - | 408 | A |
| (2) Pancreatic cancer tissue DNA | TCGA | - | 173 | B |
| (3) Liver cancer tissue DNA | TCGA | - | 460 | C |
| | GEO(GSE32079) | | | |
| (4) Lung cancer tissue DNA | TCGA | - | 832 | D |
| (5) Ovarian cancer tissue DNA | TCGA | - | 95 | E |
| | GEO(GSE51820) | | | |
| (6) Normal leukocyte DNA | GEO(GSE87571) | - | 732 | F |

### (Selection step of biomarker and production step of biomarker set)

In the same manner as in Example 1, 22 biomarkers were selected for one group from the collected DNA methylation data. In addition, 132 biomarkers of 22 pieces/group × 6 groups = 132 pieces were produced as a biomarker set for examining the presence or absence of cancer in various types of organs at once.

### (Performance evaluation of biomarker set)

In the same manner as in Example 1, after training the machine learning model SVM with the sample group used for the selection of the biomarker (that is, the methylation degree data group used for the selection of 132 biomarkers), it was estimated which organ the cancer tissue DNA of each methylation degree data of the sample group not used for the selection of the biomarker was derived from among (1) a colorectal cancer tissue, (2) a pancreatic cancer tissue, (3) a liver cancer tissue, (4) a lung cancer tissue, and (5) an ovarian cancer tissue. In the same manner as in Examples 1 and 2, the performance evaluation was shown by the estimated correct answer rate for each cancer type.

The correct answer rates of the biomarker sets selected in Comparative Example 1 were 88% for colorectal cancer, 81% for pancreatic cancer, 83% for liver cancer, 76% for lung cancer, and 72% for ovarian cancer (see Figs. 4 and 5).

### (Comparison between Example 1 and Comparative Example 1)

Fig. 4 is comparison of the results (correct answer rates) of the performance evaluation of the biomarker sets selected in Example 1 and Comparative Example 1. The biomarker set of Example 1 consists of 126 biomarkers of 14 pieces/group × 9 groups = 126 pieces, and the biomarker set of Comparative Example 1 consists of 132 biomarkers of 22 pieces/group × 6 groups = 132 pieces.

As shown in Fig. 4, it can be seen that, in each cancer type, the biomarker set selected in Example 1 has the same or higher correct answer rate than that of Comparative Example 1, although the number of biomarkers of Example 1 is smaller than that of Comparative Example 1.

Therefore, it can be seen that, the biomarker set consists of biomarkers selected after classifying sample data of cancer type of at least one organ (methylation degree data group) based on information on the tissue type can detect the cancer of various organs with higher accuracy than the biomarker set consists of biomarkers selected without classification based on information on the tissue type.

### (Comparison between Example 2 and Comparative Example 1)

Fig. 5 is comparison of the results (correct answer rates) of the performance evaluation of the biomarker sets selected in Example 2 and Comparative Example 1. The biomarker set of Example 2 consists of 132 biomarkers of 12 pieces/group × 11 groups = 132 pieces, and the biomarker set of Comparative Example 1 consists of 132 biomarkers of 22 pieces/group × 6 groups = 132 pieces. Therefore, both the biomarker sets consist of the same number of biomarkers.

As shown in Fig. 5, it can be seen that, in all cancer types, the biomarker set selected in Example 2 has a higher correct answer rate than the biomarker set selected in Comparative Example 1.

Therefore, it can be seen that, the biomarker set consists of biomarkers selected after classifying sample data of cancer type of at least one organ (methylation degree data group) based on information on the tissue type can detect the cancer of various organs with higher accuracy than the biomarker set consists of biomarkers selected without classification based on information on the tissue type.

In addition, it can be seen that even for cancer of an organ having no information on tissue type, cancer tissue DNA can be detected with high accuracy, and the presence or absence of cancer can be diagnosed.

### [Example 3]

### (Collection step of methylation degree data)

As shown in Table 4, DNA methylation degree data (sample) derived from each of (1) a colorectal cancer tissue, (2) a pancreatic cancer tissue, (3) a liver cancer tissue, (4) a lung cancer tissue, (5) an ovarian cancer tissue, and (6) a non-cancer tissue (normal leukocyte) was collected from TCGA and/or GEO of NCBI. The DNA methylation degree data consists of a position of one or more methylated sites in the entire genome region, a methylation degree of the methylated site, and information on whether or not suffering liver cirrhosis and chronic hepatitis correlated with liver cancer (3). The cancer tissue DNAs (1) to (5) of each organ in which cancer occurs are samples derived from each cancer patient, and the non-cancer tissue DNA (6) is a sample derived from a healthy subject.

It is noted that the methylation degree of all the data is measured using a microarray Infinium Human Methylation 450 of Illumina, Inc. This microarray can measure the methylation degree of about 450,000 cytosines. In the same manner as in Example 1, all cytosine bases measured by Infinium Human Methylation 450 and the methylation degree of these cytosine bases were used for the "position of one or more methylated sites in the entire genome region" and the "methylation degree of the methylated site" of the DNA methylation degree data.

### (Classification step of data)

Next, based on the information on whether or not suffering liver cirrhosis and chronic hepatitis, the sample of (3) liver cancer tissue DNA was further subdivided and classified into liver cirrhosis-affected, chronic hepatitis-affected, or neither-affected. On the other hand, the other cancers that have no information on whether or not suffering liver cirrhosis or the like were not classified. As a result, the group of cancer tissue DNAs of the organ in which cancer occurred (1) to (6) was subdivided into a total of 8 groups of A to H. The number of samples in each group is as shown in Table 4.

The DNA methylation data (sample) of each group was randomly divided into two equal parts, one sample group was used as training data of a machine learning model used for selection of a biomarker, production of a set of the biomarkers, and performance evaluation of the produced biomarker set, and the other sample group was used for performance evaluation of the produced biomarker.

**[Table 4]**

| Organ in which cancer occurred (cancer tissue DNA) | Database/GEO number | Classification based on other factors | Number of samples | Group |
|---|---|---|---|---|
| (1) Colorectal cancer tissue DNA | TCGA | - | 408 | A |
| (2) Pancreatic cancer tissue DNA | TCGA | - | 173 | B |
| (3) Liver cancer tissue DNA | | Liver fibrosis-affected | 5 | C |
| | TCGA | Chronic hepatitis-affected | 12 | D |
| | GEO(GSE60753) | | | |
| | | Neither-affected | 34 | E |
| (4) Lung cancer tissue DNA | TCGA | - | 468 | F |
| (5) Ovarian cancer tissue DNA | TCGA | - | 63 | G |
| | GEO(GSE51820) | | | |
| (6) Normal leukocyte DNA | GEO(GSE87571) | - | 732 | H |

### (Selection step of biomarker and production step of biomarker set)

In the same manner as in Example 1, 12 biomarkers were selected for one group from the collected DNA methylation data. The "position of DNA and methylation degree of DNA of A group" selected in the A group is selected as a biomarker for detecting whether or not the colorectal cancer tissue DNA is present, the "position of DNA and methylation degree of DNA of B group" selected in the B group is selected as a biomarker for detecting whether or not the pancreatic cancer tissue DNA is present, the "position of DNA and methylation degree of DNA of C group to E group" selected in the C group to E group is selected as a biomarker for detecting whether or not the liver cancer tissue DNA is present, the "position of DNA and methylation degree of DNA of F group" selected in the F group is selected as a biomarker for detecting whether or not the lung cancer tissue DNA is present, the "position of DNA and methylation degree of DNA of G group" selected in the G group is selected as a biomarker for detecting whether or not the ovarian cancer tissue DNA is present, the "position of DNA and methylation degree of DNA of H group" selected in the H group is selected as a biomarker for detecting whether or not the normal leukocyte DNA is present.

In addition, 96 biomarkers of 12 pieces/group × 8 groups = 96 pieces were produced as a biomarker set for examining the presence or absence of cancer in various types of organs at once.

### (Performance evaluation of biomarker set)

In the same manner as in Example 1, after training the machine learning model SVM with the sample group used for the selection of the biomarker (that is, the methylation degree data group used for the selection of 96 biomarkers), it was estimated which organ the cancer tissue DNA of each methylation degree data of the sample group not used for the selection of the biomarker was derived from among (1) a colorectal cancer tissue, (2) a pancreatic cancer tissue, (3) a liver cancer tissue, (4) a lung cancer tissue, and (5) an ovarian cancer tissue. In the same manner as in Example 1, the performance evaluation was shown by the estimated correct answer rate for each cancer type.

The correct answer rates of the biomarker sets selected in Example 3 were 88% for colorectal cancer, 83% for pancreatic cancer, 90% for liver cancer, 85% for lung cancer, and 84% for ovarian cancer (see Fig. 6).

### [Comparative Example 2]

### (Collection step of methylation degree data/classification step of data)

In the collection of the methylation degree data, the collection step of the methylation degree data and the classification step of data were performed in the same manner as in Examples 3, except that the information on whether or not suffering liver cirrhosis and chronic hepatitis correlated with liver cancer was not acquired in any cancer type. As a result, the group of cancer tissue DNAs of the organ in which cancer occurred (1) to (6) was classified into a total of 6 groups A to F without being subdivided. The number of samples in each of the groups A to F is as shown in Table 5.

**[Table 5]**

| Organ in which cancer occurred (cancer tissue DNA) | Database/GEO number | Classification based on other factors | Number of samples | Group |
|---|---|---|---|---|
| (1) Colorectal cancer tissue DNA | TCGA | - | 408 | A |
| (2) Pancreatic cancer tissue DNA | TCGA | - | 173 | B |
| (3) Liver cancer tissue DNA | TCGA | Liver fibrosis-affected | 5 | C |
| | | Chronic hepatitis-affected | 12 | D |
| | GEO(GSE60753) | | | |
| | | Neither-affected | 34 | E |
| (4) Lung cancer tissue DNA | TCGA | - | 468 | F |
| (5) Ovarian cancer tissue DNA | TCGA | - | 63 | G |
| | GEO(GSE51820) | | | |
| (6) Normal leukocyte DNA | GEO(GSE87571) | - | 732 | H |

### (Selection step of biomarker and production step of biomarker set)

In the same manner as in Example 3 (that is, Example 1), 22 biomarkers were selected for one group from the collected DNA methylation data. In addition, 96 biomarkers of 16 pieces/group × 6 groups = 96 pieces were produced as a biomarker set for examining the presence or absence of cancer in various types of organs at once.

### (Performance evaluation of biomarker set)

In the same manner as in Example 1, after training the machine learning model SVM with the sample group used for the selection of the biomarker (that is, the methylation degree data group used for the selection of 96 biomarkers), it was estimated which organ the cancer tissue DNA of each methylation degree data of the sample group not used for the selection of the biomarker was derived from among (1) a colorectal cancer tissue, (2) a pancreatic cancer tissue, (3) a liver cancer tissue, (4) a lung cancer tissue, and (5) an ovarian cancer tissue. In the same manner as in Examples 1 and 2, the performance evaluation was shown by the estimated correct answer rate for each cancer type.

The correct answer rates of the biomarker sets selected in Comparative Example 2 were 83% for colorectal cancer, 81% for pancreatic cancer, 82% for liver cancer, 86% for lung cancer, and 84% for ovarian cancer (see Fig. 6).

### (Comparison between Example 3 and Comparative Example 2)

Fig. 6 is comparison of the results (correct answer rates) of the performance evaluation of the biomarker sets selected in Example 3 and Comparative Example 2. The biomarker set of Example 3 consists of 96 biomarkers of 12 pieces/group × 8 groups = 96 pieces, and the biomarker set of Comparative Example 2 consists of 96 biomarkers of 16 pieces/group × 6 groups = 96 pieces. Therefore, both the biomarker sets consist of the same number of biomarkers.

As shown in Fig. 6, it can be seen that, in each cancer type, the biomarker set selected in Example 3 has the same or higher correct answer rate than the biomarker set selected in Comparative Example 2.

Therefore, it was seen that, the biomarker set consists of biomarkers selected after classifying sample data of cancer type of at least one organ (methylation degree data group) based on information on whether or not suffering liver cirrhosis and chronic hepatitis correlated with liver cancer can detect the cancer of various organs with high accuracy as the biomarker set consists of biomarkers selected without classification based on information on liver cirrhosis or the like.

In addition, it was seen that as a colorectal cancer or a pancreatic cancer, even for cancer of an organ having no information on the presence or absence of the diseases other than cancer, cancer tissue DNA can be detected with high accuracy, and the presence or absence of cancer can be diagnosed.

The biomarker set designed in the present invention can be used for cancer screening.

## Claims

1. A method for producing a biomarker set, which is a method for producing a biomarker set for use in cancer screening in which cancers of a plurality of types of organs are included as cancer to be diagnosed, the method comprising:
a collection step of collecting a plurality of DNA methylation degree data including (1) a position of one or more methylated sites in a whole genome region, (2) methylation degrees of the methylated sites, and (3) information on a tissue type, according to types of the cancer, which are a plurality of known DNA methylation degree data acquired from a biological specimen derived from a patient suffering from the cancer to be diagnosed;
a classification step of classifying the plurality of the DNA methylation degree data collected according to the types of the cancer into one or more groups based on the information on the tissue type;
an extraction step of selecting any one predetermined group from all classified groups, performing two group comparison between the predetermined group and the other group, and extracting information on a DNA position recognized to have a significant difference between both groups and a methylation state of both groups at the position;
a selection step of selecting, from the information on both groups in which the significant difference is recognized, a biomarker which satisfies a predetermined condition, as a biomarker of a cancer type to which the predetermined group belongs; and
a step of repeating the extraction step and the selection step until the biomarker is selected for all classified groups, to produce a biomarker set composed of all selected biomarkers,
wherein the information on the tissue type is included in the DNA methylation degree data of at least one or more types of the cancer.

2. The method according to claim 1,
wherein, in the classification step, in a case where the plurality of the DNA methylation degree data have information on two or more tissue types, the plurality of the DNA methylation degree data are classified into two or more groups, or in a case where the plurality of the DNA methylation degree data have information on only one of the tissue type or have no information on the tissue type, the plurality of the DNA methylation degree data are classified into one group.

3. The method according to claim 1,
wherein, in the extraction step, determination of the significant difference is performed based on (1) a p-value in a statistical test or (2) a difference between median values or between average values in distribution of the methylation degree.

4. The method according to any one of claims 1 to 3,
wherein an ovarian cancer, a lung cancer, and a liver cancer are included as the cancer to be diagnosed, and
the information on the tissue type collected in the collection step is serous carcinoma, clear cell carcinoma, endometrioid carcinoma, and mucinous carcinoma, which are tissue types of the ovarian cancer, adenocarcinoma, squamous cell carcinoma, large cell carcinoma, and small cell carcinoma, which are tissue types of the lung cancer, and hepatocellular carcinoma and intrahepatic cholangiocarcinoma, which are tissue types of the liver cancer.

5. A method for producing a biomarker set, which is a production method of a biomarker set for producing a biomarker set for use in cancer screening in which cancers of a plurality of types of organs are included as cancer to be diagnosed, the method comprising:
a collection step of collecting a plurality of DNA methylation degree data including (1) a position of one or more methylated sites in a whole genome region, (2) methylation degrees of the methylated sites, and (3) information on whether or not suffering from one or more diseases other than cancer, according to types of the cancer, which are a plurality of known DNA methylation degree data acquired from a biological specimen derived from a patient suffering from the cancer to be diagnosed;
a classification step of classifying the plurality of the DNA methylation degree data collected according to the types of the cancer into one or more groups based on the information on whether or not suffering from the one or more diseases other than cancer;
an extraction step of selecting any one predetermined group from all classified groups, performing two group comparison between the predetermined group and the other group, and extracting information on a DNA position recognized to have a significant difference between both groups and a methylation state of both groups at the position;
a selection step of selecting, from the information on both groups in which the significant difference is recognized, a biomarker which satisfies a predetermined condition, as a biomarker of a cancer type to which the predetermined group belongs; and
a step of repeating the extraction step and the selection step until the biomarker is selected for all classified groups, to produce a biomarker set composed of all selected biomarkers,
wherein the information on whether or not suffering from the one or more diseases other than cancer is included in the DNA methylation degree data of at least one or more types of the cancer.

6. The method according to claim 5,
wherein, in the classification step, in a case where the plurality of the DNA methylation degree data have the information on whether or not suffering from the one or more diseases other than cancer, the plurality of the DNA methylation degree data are classified into two or more groups, or in a case where the plurality of the DNA methylation degree data have no information on whether or not suffering from the diseases other than cancer, the plurality of the DNA methylation degree data are classified into one group.

7. The method according to claim 5,
wherein, in the extraction step, determination of the significant difference is performed based on (1) a p-value in a statistical test or (2) a difference between median values or between average values in distribution of the methylation degree.

8. The method according to any one of claims 5 to 7,
wherein a liver cancer is included as the cancer to be diagnosed, and
the information on whether or not suffering from the one or more diseases other than cancer collected in the collection step is whether or not suffering from liver cirrhosis and whether or not suffering from chronic hepatitis, which are highly correlated with the liver cancer.
